(19) Europäisches Patentamt
European Patent Office
Office européen des brevets

(11) **EP 1 172 092 B1**

(12) **FASCICULE DE BREVET EUROPEEN**

(45) Date de publication et mention de la délivrance du brevet:
**15.09.2004 Bulletin 2004/38**

(51) Int Cl.[7]: **A61K 7/48**

(21) Numéro de dépôt: **01401747.9**

(22) Date de dépôt: **29.06.2001**

(54) **Compositions comprenant au moins un filtre UV et un sel de flavylium non substitué en position 3 pour la coloration de la peau**

Hautfärbemittel enthaltend Flavylium-Salz Verbindungen, die in Position 3 unsubstituierte sind, und mindestens einen UV Filter

Compositions for coloring the skin containing at least one UV filter and flavylium salt compounds unsubstituted in position 3

(84) Etats contractants désignés:
**AT BE CH CY DE DK ES FI FR GB GR IE IT LI LU MC NL PT SE TR**

(30) Priorité: **12.07.2000 FR 0009117**

(43) Date de publication de la demande:
**16.01.2002 Bulletin 2002/03**

(73) Titulaire: **L'OREAL**
**75008 Paris (FR)**

(72) Inventeurs:
- **Candau, Didier**
**F-91570 Bievres (FR)**
- **Forestier, Serge**
**F-77410 Claye Souilly (FR)**

(74) Mandataire: **Miszputen, Laurent**
**L'OREAL - D.I.P.I.**
**25-29 Quai Aulagnier**
**92600 Asnières (FR)**

(56) Documents cités:
**EP-A- 0 024 731** **FR-A- 2 757 383**

- **DATABASE WPI Week 199929 Derwent Publications Ltd., London, GB; AN 1999-338248 XP002164085 & CN 1 209 992 A (ZHANG)**
- **DATABASE WPI Week 198828 Derwent Publications Ltd., London, GB; AN 1988-195805 XP002164086 & JP 63 135310 A (LION CORP)**
- **DATABASE WPI Week 198714 Derwent Publications Ltd., London, GB; AN 1987-099079 XP002164100 & JP 62 048611 A (SHISEIDO)**
- **La recherche additionnelle effectuée n'a pas permis de révéler de documents supplémentaires.**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 172 092 B1

Printed by Jouve, 75001 PARIS (FR)

## Description

La présente invention se rapporte à des compositions cosmétiques et/ou dermatologiques destinées à la coloration artificielle de la peau, caractérisée par le fait qu'elle comprend, dans un support cosmétiquement acceptable, un composé capable de filtrer le rayonnement ultra violet et au moins un composé du type sel de flavylium, non substitué en position 3, et substitué par au moins un radical hydroxyle ou alcoxy, obtenu par voie de synthèse ou à partir d'un extrait végétal le contenant ou bien encore à partir d'un extrait végétal enrichi le contenant.

L'invention concerne également les applications de ces compositions à la coloration de la peau.

De nos jours, il est important d'avoir bonne mine et une peau bronzée est toujours signe de bonne santé. Cependant, le bronzage naturel n'est pas toujours souhaitable dans la mesure où il nécessite des expositions prolongées aux rayonnements UV, en particulier aux rayonnements UV-A qui provoquent le brunissement de la peau mais en contrepartie sont susceptibles d'induire une altération de celle-ci, notamment dans le cas d'une peau sensible ou d'une peau continuellement exposée au rayonnement solaire. Il est donc souhaitable de trouver une alternative au bronzage naturel qui soit compatible avec les exigences de telles peaux.

La plupart des produits cosmétiques destinés au bronzage artificiel de la peau sont à base composés mono ou polycarbonylés permettant, par interaction avec les acides aminés de la peau, la formation de produits colorés.

A cet effet, on sait que la dihydroxyacétone, ou DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV.

Un inconvénient de la DHA est la lenteur avec laquelle la coloration se développe : il faut en effet compter plusieurs heures (3 à 5 heures en général) pour que soit révélée la coloration..

De plus, la DHA présente une fâcheuse tendance, plus ou moins prononcée selon la nature du milieu dans lequel elle est formulée, à se dégrader au cours du temps, cette dégradation se traduisant généralement à terme par un jaunissement non souhaitable des compositions qui la contiennent. Un tel phénomène fait que l'activité de la DHA, et en particulier son aptitude à colorer la peau, peut être diminuée au moment de l'application de ces compositions sur la peau. Ainsi, l'intensité de la coloration, la tenue de la coloration dans le temps obtenues sur la peau peuvent apparaître comme encore insuffisantes.

Ainsi, on est toujours à la recherche de nouveaux composés et de nouvelles compositions permettant de conférer artificiellement à la peau une coloration proche du bronzage naturel d'une manière simple, efficace, rapide et sans risque. On cherche également à obtenir une coloration plus homogène et plus tenace.

Les colorants anthocyaniques sont connus depuis longtemps comme colorants alimentaires et pharmaceutiques. Ces anthocyanes sont présents dans la nature sous forme d'hétérosides appelés anthocyanosides et de génines, appelées anthocyanidines. Ces anthocyanes sont des dérivés du phényl-2-benzopyrylium ou flavylium et sont notamment présents dans la plante sous forme de sels. Les anthocyanes sont des composés de teinte rouge, violette ou bleue qui colorent généralement les fleurs, les fruits et parfois les feuilles. La couleur observée dépend à la fois de la structure de la génine majoritaire et des conditions du milieu où se trouvent les colorants anthocyaniques.

Or, à la suite d'importantes recherches menées dans le domaine de la coloration artificielle de la peau, la Demanderesse a maintenant découvert que l'association au moins un agent filtrant les radiations UV, organique ou minéral et d'un ou plusieurs composés particuliers du type sel de flavylium non substitué en position 3 permettait de conférer immédiatement après l'application sur la peau du produit une coloration artificielle proche du bronzage naturel. De plus, ladite association permet d'obtenir par rapport à un autobronzant du type carbonylé tel que la DHA une nuance plus homogène et plus tenace.

La présente invention a donc pour objet une nouvelle composition cosmétique et/ou dermatologique, destinée à la coloration artificielle de la peau proche du bronzage naturel, caractérisée par le fait qu'elle comprend, dans un support cosmétiquement acceptable, au moins un agent filtrant les radiations UV, organique ou minéral et au moins un composé du type sel de flavylium, non substitué en position 3, et substitué par au moins un radical hydroxyle ou alcoxy, obtenu par voie de synthèse ou à partir d'un extrait végétal le contenant ou bien encore à partir d'un extrait végétal enrichi.

La présente invention a encore pour objet l'utilisation nouvelle de l'association d'au moins un agent filtrant les radiations UV, organique ou minéral et d'au moins un composé du type sel de flavylium, non substitué en position 3, et substitué par au moins un radical hydroxyle ou alcoxy, obtenu par voie de synthèse, ou à partir d'un extrait végétal, ou d'un extrait végétal enrichi, dans des compositions cosmétiques et/ou dermatologiques dans le but de conférer à la peau une coloration artificielle proche du bronzage naturel.

La présente invention a également pour objet un procédé de coloration artificielle proche du bronzage naturel de la peau, caractérisé en ce qu'il consiste à appliquer sur celle-ci une quantité efficace de l'association d'au moins un agent filtrant les radiations UV, organique ou minéral et d'au moins un composé du type sel de flavylium, non substitué en position 3, et substitué par au moins un radical hydroxyle ou alcoxy, obtenu par voie de synthèse, ou à

partir d'un extrait végétal, ou d'un extrait végétal enrichi ; à partir d'une composition cosmétique.

Les compositions et les utilisations conformes à l'invention permettent d'obtenir une coloration artificielle proche du bronzage naturel en un laps de temps court. Ainsi, on obtient une coloration immédiate qui permet une visualisation de l'application et par conséquent une meilleure homogénéité dans l'étalement de la composition sur la peau et donc de la coloration qui en résulte. De plus, la coloration artificielle obtenue sur la peau selon l'invention est proche du bronzage naturel.

Au sens de la présente invention, on entendra, par « composition destinée à la coloration artificielle de la peau », une formulation ayant une affinité particulière pour la peau lui permettant de conférer à cette dernière une coloration durable, non-couvrante (à savoir n'ayant pas tendance à opacifier la peau) et qui ne s'élimine ni à l'eau ni à l'aide d'un solvant, et qui résiste à la fois au frottement et au lavage par une solution contenant des tensioactifs. Une telle coloration durable se distingue donc de la coloration superficielle et momentanée apportée par exemple par un produit de maquillage.

D'autres caractéristiques, aspects et avantages de la présente invention apparaîtront à la lecture de la description détaillée qui va suivre.

Les compositions conformes à la présente invention conduisent en général au bout de 30 minutes après application sur une peau claire à raison de $2mg/cm^2$ à un assombrissement caractérisé dans le système de mesure colorimétrique (L*, a*, b*) par un $\Delta L^*$ allant de -0,5 à - 20. De façon plus préférentielle, $\Delta L^*$ variera de - 0,5 à - 15.

Les compositions conformes à la présente invention apportent au bout de 30 minutes après application sur la peau à raison de $2mg/cm^2$ une coloration sur une peau claire qui est définie dans le système de mesure colorimétrique (L*, a*, b*), par un rapport $\Delta a^*/\Delta b^*$ allant de 0,5 à 3 et encore plus particulièrement de 0, 8 à 2.

Selon la présente invention, on entend « par peau claire », des peaux non bronzées dont on peut définir les caractéristiques colorimétriques par leur angle ITA tel que défini dans la publication de A. Chardon et al. « Skin Colour Typology and Suntanning Pathways » et présentée au $16^{th}$ IFSCC congress oct 8-10 1990 de New-York, et in *Int. J. Cosm Sci* **13** 191-208 (1991). Les peaux claires telles que définies dans cette classification ont un angle ITA compris entre 35 et 55.

Dans le système de mesure colorimétrique (L*, a*, b*) :

L* représente la luminance ou clarté, a* représente l'axe rouge-vert (-a*= vert, +a*= rouge) et b* représente l'axe jaune-bleu (-b*=bleu, +b*=jaune). Ainsi, a* et b* expriment la nuance de la peau.

$\Delta L^*$ traduit l'assombrissement de la couleur : plus le $\Delta L^*$ est négatif, plus la couleur s'est assombrie avec :

$$\Delta L^* = L^* \text{ peau non colorée} - L^* \text{ peau colorée}$$

Le rapport $\Delta a^*/\Delta b^*$ traduit l'équilibre rouge/jaune et donc la nuance avec :

$$\Delta a^* = a^* \text{ peau non colorée} - a^* \text{ peau colorée}$$

$$\Delta b^* = b^* \text{ peau non colorée} - b^* \text{ peau colorée}$$

Parmi les composés du type sel de flavylium non substitués en position 3 conformes à l'invention, on utilisera de préférence ceux répondant à la formule (I) suivante :

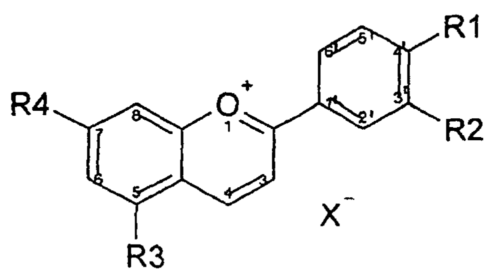

(I)

formule (I) dans laquelle :

- $R_1$ désigne un radical OH ou alcoxy en $C_1$-$C_8$, linéaire ou ramifié, saturé ou insaturé,
- $R_2$, $R_3$, $R_4$, identiques ou différents, désignent H ou $R_1$, étant entendu qu'au moins un des radicaux $R_1$ à $R_4$ désigne OH,
- $X^-$ est un anion organique ou minéral et de préférence un dérivé d'acide minéral tel que par exemple un halogénure comme bromure ou chlorure ou bien un dérivé d'un acide organique comme par exemple acétate, borate, citrate, tartrate, lactate, bisulfate, sulfate, phosphate.

Les composés de formule (I) particulièrement préférés selon la présente invention sont choisis dans le groupe de ceux pour lesquels, dans la formule (I), $R_1$ désigne OH ou $OCH_3$.
On peut citer notamment parmi eux, les chlorures des composés suivants :

- 4', 5, 7-trihydroxyflavylium, communément dénommé "chlorure d'apigéninidine",
- 3', 4', 7- trihydroxyflavylium,
- 4'-hydroxyflavylium,
- 4', 7-dihydroxyflavylium,
- 3', 4'-dihydroxyflavylium,
- 3', 4'-dihydroxy-7-méthoxy-flavylium,
- 3',4', 5, 7-tétrahydroxyflavylium,
- 3', 4', 5', 5, 7-pentahydroxyflavylium.

Parmi ces composés, le chlorure d'apigéninidine (chlorure de 4', 5, 7-trihydroxyflavylium) et le chlorure de 3', 4', 7- trihydroxyflavylium sont encore plus particulièrement préférés.
Une forme particulière de l'invention consiste à utiliser le chlorure d'apigéninidine sous forme d'un extrait végétal, aisément préparé par extraction, et isolé, à partir de feuilles de Sorghum caudatum selon les procédés décrits dans les brevets CN 1064284A et CN1035512C ou toutes autres variantes de ces procédés .
Il peut aussi être extrait des tiges, des graines, ou des feuilles de Sorghum Bicolor, des pétales de Gesneria Fulgens, ainsi que des espèces Blechum Procerum et Sorgho en association avec du Colletotrichum Graminicola.
Une forme particulièrement préférée de l'invention est un extrait provenant des feuilles de Sorghum Bicolor obtenu par une extraction hydro-alcoolique en milieu acide à une température d'extraction allant de 30 à 40°C avec un rapport volume de solvant / masse de feuilles de Sorghum Bicolor allant de 10 à 30. Ledit extrait végétal de Sorghum titre environ 0,05 à 50% en poids de chlorure d'apigéninidine.
Les composés du type sel de flavylium, non substitués en position 3, et substitués par au moins un radical hydroxyle ou alcoxy, selon l'invention, peuvent être facilement obtenus par voie de synthèse, et à faible coût, notamment par la méthode bien connue de R. Robinson et D. Pratt J. Chem. Soc. 745 (1923). Ladite méthode implique de condenser une orthohydroxybenzaldéhyde ou ses dérivés de substitution sur une acétophénone ou ses dérivés de substitution, pour obtenir, en choisissant les substituants, les composés de formule (I) désirés.
En prenant comme exemple le chlorure d'apigéninidine (chlorure de 4', 5, 7-trihydroxyflavylium), le schéma de synthèse (i) peut être le suivant :

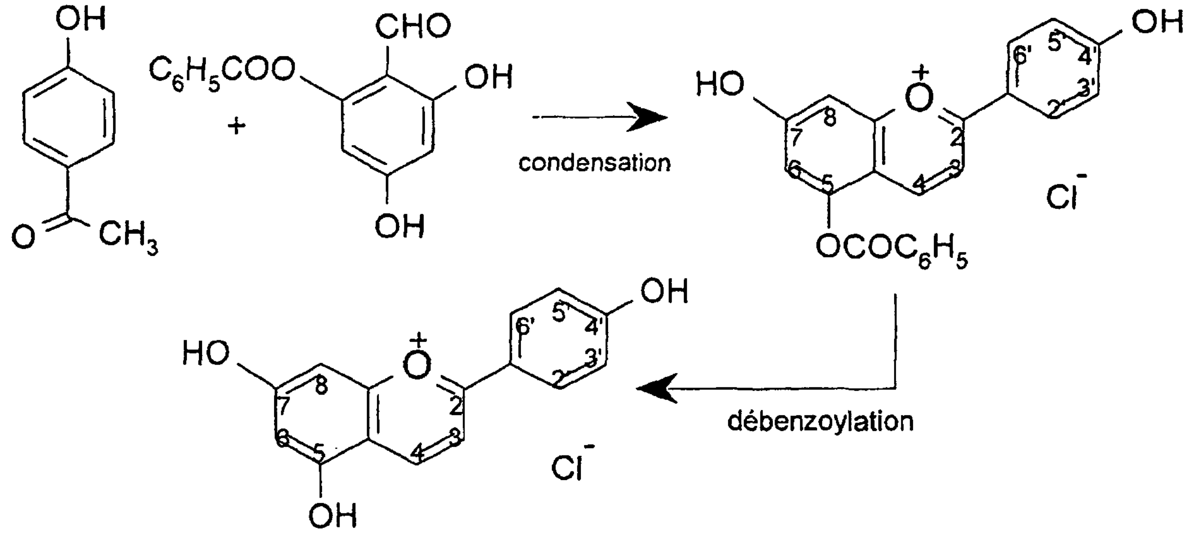

En prenant comme exemple le chlorure de 3', 4', 7- trihydroxyflavylium, le schéma de synthèse (ii) peut être le suivant :

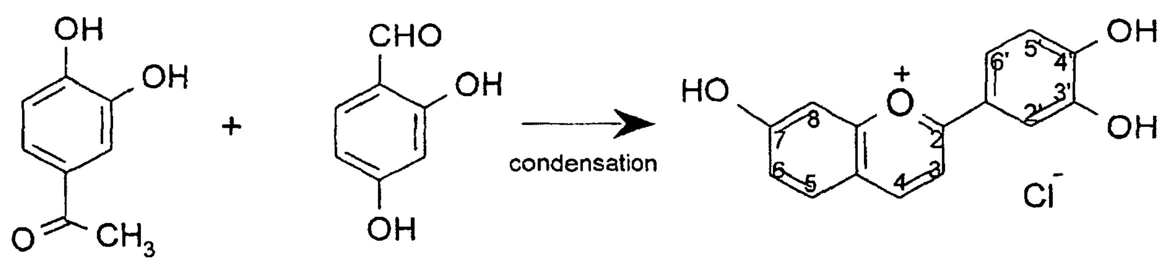


Diverses voies de synthèses, bien connues dans l'art antérieur, conduisent à l'apigéninidine.
Une méthode consiste, par exemple, à préparer, dans une première étape, la triméthylapigéninidine, par condensation du 4,6-diméthoxy-2-hydroxybenzaldéhyde commercial sur la 4-méthoxyacétophénone commerciale à 0°C en milieu éther anhydre, et saturation par HCl anhydre, pour obtenir après filtration un précipité rouge-orangé de triméthylapigéninidine. Dans une seconde étape, à hydrolyser la triméthylapigéninidine obtenue à l'étape précédente en chlorure d'apigéninidine, la réaction s'effectuant en milieu HI et phénol et AgCl en solution dans le méthanol. Une telle méthode de synthèse est décrite par R. Robinson et A. Robertson dans J. Chem. Soc. 1951 (1926) et 2196 (1927).
Une autre méthode consiste à condenser le 2, 4, 6-trihydroxybenzaldéhyde sur la 4-hydroxyacétophénone à 0°C en milieu solvant anhydre (acétate d'éthyle par exemple), et saturation par HCl anhydre, pour obtenir le chlorure d'apigéninidine. Une telle méthode est décrite par R. Robinson et A. Robertson dans J. Chem. Soc. 1528 (1928).
Une autre méthode de préparation du chlorure d'apigéninidine consiste à réduire une flavone, la naringénine, ou son dérivé triacétylé, par $NaBH_4$, puis à oxyder le produit obtenu par le chloranil (tétrachloro-1,4-benzoquinone). Ladite méthode est décrite par J. G. Sweeny et G. A. Iacobucci dans la revue Tetrahedron **33** 2923-2927 (1977).
La méthode la plus particulièrement préférée, selon la présente invention, consiste à condenser le 2,4-dihydroxy-6-benzoyloxybenzaldéhyde sur la 4-hydroxyacétophénone à 0°C en milieu acétate d'éthyle anhydre, à saturer par HCl anhydre, puis à débenzoyler le produit obtenu par la soude, afin d'obtenir le chlorure d'apigéninidine avec un rendement élevé, suivant le schéma (i) décrit ci-dessus. Ladite méthode est décrite par R. Robinson et J. C. Bell dans J. Chem. Soc. 813 (1934).
La concentration en composé du type sel de flavylium, tel que décrit selon la présente invention, varie de préférence de environ 0,0001 à 10%, et encore plus préférentiellement de 0,001 à 5% en poids, par rapport au poids total de la composition.
Les agents filtrant le rayonnement ultra violet peuvent être choisis parmi les filtres UV organiques ou les agents filtrant les radiations UV minéraux.
Les filtres UV organiques conformes à l'invention peuvent être hydrosolubles, liposolubles ou insolubles dans les solvants usuels cosmétiques. Ils sont choisis notamment parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP933376 ; les dérivés de la benzophénone ; les dérivés de β, β'-diphénylacrylate, les dérivés de benzotriazole, les dérivés de benzimidazole ; les imadazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE19726184 et EP893119 ; les polymères filtres et silicones filtres tels que ceux décrits notamment dans la demande WO93/04665 ; les dimères dérivés d'α-alkylstyrène tels que ceux décrits dans la demande de brevet DE19855649 ; les dérivés de 4,4-diarylbutadiène tels que ceux décrits dans les demandes de brevet EP0967200 et DE19755649.
Comme exemples de filtres organiques, on peut citer désignés ci-dessus sous leur nom INCI :

Dérivés de l'acide para-aminobenzoique :

- PABA,
- Ethyl PABA,
- Ethyl Dihydroxypropyl PABA,
- Ethylhexyl Diméthyl PABA vendu notamment sous le nom « ESCALOL 507 » par ISP,

- Glyceryl PABA,
- PEG-25 PABA vendu sous le nom « UVINUL P25 » par BASF,

Dérivés salicyliques :

- Homosalate vendu sous le nom « EUSOLEX HMS » par RONA/EM INDUSTRIES,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate, vendu sous le nom « NEO HELIOPAN TS » par HAARMANN et REIMER,

Dérivés du dibenzoylméthane :

- Butyl Methoxydibenzoylmethane vendu notamment sous le nom commercial « PARSOL 1789 » par HOFFMANN LA ROCHE,
- Isopropyl Dibenzoylmethane,

Dérivés cinnamiques :

- Ethylhexyl Methoxycinnamate vendu notamment sous le nom commercial « PARSOL MCX » par HOFFMANN LA ROCHE,
- Isopropyl Methoxy cinnamate,
- Isoamyl Methoxy cinnamate vendu sous le nom commercial « NEO HELIOPAN E 1000 » par HAARMANN et REIMER,
- Cinoxate,
- DEA Methoxycinnamate,
- - Diisopropyl Methylcinnamate,
- Glyceryl Ethylhexanoate Dimethoxycinnamate

Dérivés de β,β'-diphénylacrylate :

- Octocrylene vendu notamment sous le nom commercial « UVINUL N539 » par BASF,
- Etocrylene, vendu notamment sous le nom commercial « UVINUL N35 » par BASF,

Dérivés de la benzophénone :

- Benzophenone-1 vendu sous le nom commercial « UVINUL 400 » par BASF,
- Benzophenone-2 vendu sous le nom commercial « UVINUL D50 » par BASF
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5
- Benzophenone-6 vendu sous le nom commercial « HELISORB 11 » par NORQUAY
- Benzophenone-8 vendu sous le nom commercial « SPECTRA-SORB UV-24 » PAR AMERICAN CYANAMID
- Benzophenone-9 vendu sous le nom commercial« UVINUL DS-49» par BASF,
- Benzophenone-12

Dérivés du benzylidène camphre :

- 3-Benzylidene camphor fabriqué sous le nom « MEXORYL SD» par CHIMEX,
- 4-Methylbenzylidene camphor vendu sous le nom « EUSOLEX 6300 » par MERCK ,
- Benzylidene Camphor Sulfonic Acid fabriqué sous le nom « MEXORYL SL» par CHIMEX,
- Camphor Benzalkonium Methosulfate fabriqué sous le nom « MEXORYL SO » par CHIMEX,
- -Terephthalylidene Dicamphor Sulfonic Acid fabriqué sous le nom « MEXORYL SX » par CHIMEX,
- Polyacrylamidomethyl Benzylidene Camphor fabriqué sous le nom « MEXORYL SW » par CHIMEX,

Dérivés du phenyl benzimidazole :

- Phenylbenzimidazole Sulfonic Acid vendu notamment sous le nom commercial « EUSOLEX 232 » par MERCK,
- Benzimidazilate vendu sous le nom commercial commercial « NEO HELIOPAN AP » par HAARMANN et REIMER,

Dérivés de la triazine :

- Anisotriazine vendu sous le nom commercial «TINOSORB S » par CIBA GEIGY,
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,

Dérivés du phenyl benzotriazole :

- Drometrizole Trisiloxane vendu sous le nom « SILATRIZOLE » par RHODIA CHIMIE ,
- Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

Dérivés anthraniliques :

- Menthyl anthranilate vendu sous le nom commercial commercial « NEO HELIOPAN MA » par HAARMANN et REIMER,

Dérivés d'imidazolines :

- Ethylhexyl Dimethoxybenzylidene Dioxoimidazoline Propionate,

Dérivés du benzalmalonate :

- Polyorganosiloxane à fonctions benzalmalonate vendu sous la dénomination commerciale « PARSOL SLX » par HOFFMANN LA ROCHE

et leurs mélanges.

Les filtres UV organiques plus particulièrement préférés sont choisis parmi les composés suivants :

- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,.
- Benzophenone-3,
- Benzophenone-4,
- Benzophenone-5,
- 4-Methylbenzylidene camphor,
- Benzimidazilate,
- Anisotriazine,
- Ethylhexyl triazone,
- Diethylhexyl Butamido Triazone,
- Methylene bis-Benzotriazolyl Tetramethylbutylphenol,
- Drometrizole Trisiloxane,

et leurs mélanges.

Les agents filtrant minéraux sont généralement des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les agents filtrants les radiations conformes à l'invention sont généralement présents dans les compositions selon l'invention dans des proportions allant de 0,1 à 20% en poids par rapport au poids total de la composition, et de préférence allant de 0,2 à 15% en poids par rapport au poids total de la composition.

Les compositions conformes à la présente invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les $\alpha$-hydroxyacides, les agents anti-mousse, les agents hydratants, les vitamines, les agents répulsifs contre les insectes, les parfums, les conservateurs, les tensioactifs, les charges, les polymères, les propulseurs, les agents alcalinisants ou acidifiants, les colorants, ou tout autre ingrédient habituellement utilisé dans le domaine cosmétique et/ou dermatologique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en $C_{12}$-$C_{15}$ vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Selon une forme particulièrement préférée, les compositions selon l'invention contiennent au moins 5% en poids par rapport au poids de la composition d'un ou plusieurs solvants polyhydroxylés. Ces derniers peuvent être choisis parmi les glycols et les éthers de glycol comme l'éthylène glycol, le propylène glycol, le butylène glycol, le dipropylène glycol ou le diéthylène glycol. Et plus particulièrement, les compositions selon l'invention contiennent un mélange d'au moins trois solvants polyhydroxylés différents et encore plus particulièrement un mélange constitué de propylène glycol, de butylène glycol, et de dipropylène glycol.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir le ou les éventuels composés complémentaires cités ci-dessus et/ou leurs quantités de manière telle que les propriétés avantageuses attachées intrinsèquement à l'association conforme à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Des exemples concrets, mais nullement limitatifs, illustrant l'invention, vont maintenant être donnés.

## EXEMPLE 1 :

On prépare un extrait de Sorghum Bicolor titrant à 20-30% de chlorure d'apigéninidine selon le procédé de préparation suivant :
un extrait provenant des feuilles de Sorghum Bicolor est obtenu par extraction hydro-alcoolique (éthanol 95°) en milieu acide (0.2% HCl) à une température d'extraction de 35°C avec un rapport volume de solvant / masse de feuilles de Sorghum Bicolor de 15. L' extrait végétal de Sorghum est séché à l'étuve 24 h à 40°C et tamisé à 200 µm.

Le rendement de cette extraction est de 22.42% en matière colorante.
Le titre de l'extrait ainsi obtenu est de 21% en poids de chlorure d'apigéninidine.

Cet exemple vise à montrer l'intensité de la coloration obtenue avec un extrait de Sorghum Bicolor associé à un filtre UV conforme à la présente invention ainsi que la rapidité avec laquelle cette coloration se développe par rapport à une composition contenant la DHA seule à titre d'agent de coloration de la peau.

La Demanderesse a réalisé les compositions suivantes (les quantités sont exprimées en pourcentage de poids par rapport au poids total de la composition) :

Composition A (hors invention):

- Polydiméthyl/méthyl siloxane POE/POP(396/4) (OE/OP 18/18) A 10 % D5 10g
- Cyclopentadiméthylsiloxane 12.5g
- Mélange tocophérols naturels/huile de soja 0.1g
- Dihydroxyacétone (DHA) 4g
- Chlorure de sodium 2g
- Propylène glycol 23g
- Butylène glycol 5g
- Dipropylène glycol 10g
- Eau déminéralisée 32.649g
- Citrate trisodique 0.542g
- Acide citrique 0.209g

Composition B (invention)

- Polydiméthyl/méthyl siloxane POE/POP(396/4) (OE/OP 18/18) A 10 % D5 10g
- Cyclopentadiméthylsiloxane 12.5g
- Mélange tocophérols naturels/huile de soja 0.1g
- Eau déminéralisée 35.659g
- Acide benzène 1,4-di(3-méthylidène-10-camphosulfonique) 0,5g
- Chlorure de sodium 2g
- Propylène glycol 23g
- Butylène glycol 5g
- Dipropylène glycol 10g

- Extrait de Sorghum Bicolor tel que préparé ci-dessus 0.5g
- Citrate trisodique 0.535g
- Acide citrique 0.206g

**Protocole d'évaluation :**

Les compositions A et B ont été appliquées à raison de 2 mg/$cm^2$ sur une zone de 7 x 4,5 $cm^2$ délimitée sur le dos de 6. volontaires dont la couleur de peau caractérisée par l'angle ITA est compris entre 35 et 55.).

Les cinq séries de mesures colorimétriques suivantes ont été effectuées à l'aide d'un colorimètre Minolta CR-300 :

- 1°) avant application de la composition,
- 2°) 30 minutes après l'application,
- 3°) 2 heures après application.
- 4°) 4 heures après application.

Les résultats sont exprimés dans le système (L*, a*, b*) dans lequel L* représente la luminance, a* représente l'axe rouge-vert (-a* = vert, +a* = rouge) et b* représente l'axe jaune-bleu (-b* =bleu, +b* =jaune). Ainsi, a* et b* expriment la nuance de la peau.

Pour l'évaluation de l'intensité de la coloration, on s'intéresse à ΔL* qui traduit l'assombrissement de la couleur : plus ΔL* est négatif, plus la couleur s'est assombrie avec :

$$\Delta L^* = L^* \text{ peau non colorée} - L^* \text{ peau colorée}$$

Les résultats obtenus sont rassemblés dans le tableau (I) suivant :

Tableau (I) :

| | **Composition A (comparative) ΔL*** | **Composition B (invention) ΔL*** |
|---|---|---|
| 30 minutes | -0.4 | -8.2 |
| 2 heures | -1.1 | -7 |
| 4 heures | -2.5 | -6.7 |

On constate ainsi que 30 minutes après l'application, la composition A, qui contient à titre d'agent de coloration de la peau, la DHA, n'a conféré qu'une très faible coloration à la peau, puisque la DHA n'a pas encore eu le temps d'agir (ΔL* = -0.4). En revanche, la composition B selon l'invention a déjà conféré à la peau une coloration significative (ΔL* =-8.2).

## Revendications

1. Composition cosmétique et/ou dermatologique pour la coloration artificielle de la peau proche du bronzage naturel, **caractérisée par le fait qu'**elle comprend, dans un support cosmétiquement acceptable, au moins un agent filtrant les radiation UV, organique ou minéral et au moins un composé du type sel de flavylium qui répond à la formule (1) suivante :

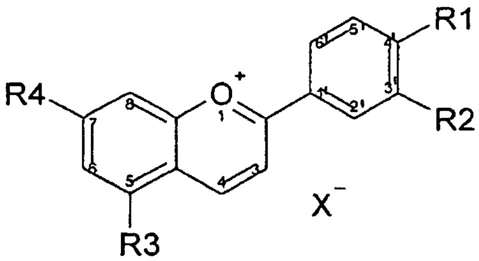

(I)

dans laquelle:

- $R_1$ désigne un radical OH ou alcoxy en $C_1$-$C_8$, linéaire ou ramifié, saturé ou insaturé,
- $R_2$, $R_3$, $R_4$, identiques ou différents, désignent H ou $R_1$, étant entendu qu'au moins un des radicaux $R_1$ à $R_4$ désigne OH,
- $X^-$ est un anion organique ou minéral et de préférence du type halogènure ou dérivé d'un acide organique,

obtenu par voie de synthèse ou à partir d'un extrait végétal le contenant ou bien encore à partir d'un extrait végétal enrichi.

**2.** Composition selon la revendication 1, **caractérisée par le fait qu'**elle conduit au bout de 30 minutes après application sur une peau claire à raison de 2mg/cm2 à un assombrissement caractérisé dans le système de mesure colorimétrique L* a* b* par un ΔL* allant de - 0,5 à -20.

**3.** Composition selon la revendication 2, où le ΔL* varie de - 0,5 à -15.

**4.** Composition selon la revendication 1 à 3, **caractérisée par le fait qu'**elle produit au bout de 30 minutes après application sur une peau claire à raison de 2mg/$cm^2$ une coloration définie dans le système colorimétrique L* a* b* par un rapport Δa*/Δb* allant de 0,3 à 3.

**5.** Composition selon la revendication 3, selon laquelle le rapport Δa*/Δb* varie de 0,8 à 2.

**6.** Composition selon la revendication 1, où dans la formule (I), $R_1$ désigne OH ou $OCH_3$.

**7.** Composition selon la revendication 5, où le composé de formule (I) est choisi parmi les chlorures des composés suivants :

- 4', 5, 7-trihydroxy sel de flavylium (chlorure d'apigéninidine),
- 3', 4', 7- trihydroxy sel de flavylium,
- 4'-hydroxy sel de flavylium,
- 4', 7-dihydroxy sel de flavylium,
- 3', 4'-dihydroxy sel de flavylium,
- 3', 4'-dihydroxy-7-méthoxy- sel de flavylium
- 3',4', 5, 7-tétrahydroxyflavinium,
- 3', 4', 5', 5, 7-pentahydroxyflavinum.

**8.** Composition selon la revendication 7, **caractérisée par le fait qu'**il s'agit du chlorure de 4', 5, 7-trihydroxy sel de flavylium sous forme pure susceptible d'être obtenu par voie de synthèse.

**9.** Composition selon la revendication 7, **caractérisée par le fait qu'**il s'agit du chlorure de 4', 5, 7-trihydroxy sel de flavylium, sous forme d'extrait végétal.

**10.** Composition selon la revendication 9, **caractérisée par le fait que** l'extrait végétal est un extrait végétal, obtenu à partir de feuilles de Sorghum caudatum, ; de tiges, de graines ou de feuilles de Sorghum Bicolor ; des pétales de Gesneria Fulgens, ainsi que des espèces Blechum Procerum et Sorgho en association avec du Colletotrichum Graminicola.

**11.** Composition selon la revendication 9, **caractérisée par le fait que** l'extrait végétal est un extrait de Sorghum

Bicolor susceptible d'être obtenu par une extraction hydro-alcoolique acide à une température d'extraction allant de 30 à 40°C avec un rapport volume de solvant / masse de feuilles de Sorghum Bicolor allant de 10 à 30.

**12.** Composition selon la revendication 11, **caractérisée par le fait que** l'extrait de Sorghum Bicolor titre de 0,05 à 50% en poids de chlorure de 4', 5, 7-trihydroxy sel de flavylium.

**13.** Composition selon l'une quelconque des revendications 1 à 11, où la concentration en composé du type sel de flavylium varie de 0,0001 à 10%

**14.** Composition selon la revendication 13, où la concentration en composé du type sel de flavylium, varie de 0,001 à 5% en poids, par rapport au poids total de la composition.

**15.** Composition selon l'une quelconque des revendications 1 à 14, où le ou les agents filtrant les radiations UV organiques sont hydrosolubles, liposolubles ou insolubles dans 35 les solvants usuels cosmétiques.

**16.** Composition selon l'une quelconque des revendications 1 à 15, où le ou les agents filtrant les radiations UV organiques sont choisis parmi les anthranilates ; les dérivés cinnamiques ; les dérivés de dibenzoylméthane ; les dérivés salicyliques, les dérivés du 40 camphre ; les dérivés de triazine ; les dérivés de la benzophénone ; les dérivés de $\beta,\beta'$-diphénylacrylate, les dérivés de benzotriazole, les dérivés de benzimidazole ; les imadazolines ; les dérivés bis-benzoazolyle ; les dérivés de l'acide p-aminobenzoïque (PABA) ; les dérivés de méthylène bis-(hydroxyphényl benzotriazole) ; les polymères filtres et silicones filtres ; les dimères dérivés d'$\alpha$-alkylstyrène ; les dérivés de 4,4-diarylbutadiène.

**17.** Composition selon la revendication 16, où le ou les agents filtrants les radiations UV organiques sont choisis parmi :

- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,.
- Benzophenone-3,
- Benzophenone-4,
- Benzophenone-5,
- 4-Methylbenzylidene camphor,
- Benzimidazilate,
- Anisotriazine,
- Ethylhexyl triazone,
- Diethylhexyl Butamido Triazone,
- Methylene bis-Benzotriazolyl Tetramethylbutylphenol,
- Drometrizole Trisiloxane,

et leurs mélanges.

**18.** Composition selon l'une quelconque des revendications 1 à 15, où le ou les agents filtrant les radiations UV minéraux sont choisis parmi les pigments ou les nanopigments d'oxydes métalliques enrobés ou non.

**19.** Composition selon la revendication 16, où le ou les agents filtrant les radiations UV minéraux sont choisis parmi les nanopigments d'oxyde de titane, de fer, de zinc, de zirconium ou de cérium, enrobés ou non.

**20.** Composition selon l'une quelconque des revendications 1 à 21, où le ou les agents filtrant les radiations UV sont présents dans des proportions allant de 0,1 à 15% en poids par rapport au poids total de la composition.

**21.** Composition selon la revendication 22, où le ou les agents filtrant les radiation UV sont présents dans des proportions allant de 0,1 à 15% en poids par rapport au poids total de la composition.

**22.** composition selon l'une quelconque des revendications 1 à 23, contenant au moins 5% en poids par rapport au poids de la composition d'un ou plusieurs solvants polyhydroxylés.

**23.** Composition selon la revendication 24, où les solvants polyhydroxylés sont choisis parmi les glycols et les éthers de glycol.

**24.** Composition selon la revendication 24 ou 25, où les solvants polyhydroxylés sont choisis parmi l'éthylène glycol, le propylène glycol, le butylène glycol, le dipropylène glycol ou le diéthylène glycol et leurs mélanges.

**25.** Composition selon l'une quelconque des revendications 24 à 26, contenant un mélange de trois solvants polyhydroxylés différents .

**26.** Composition selon la revendication 27, contenant un mélange constitué de propylène glycol, de butylène glycol, et de dipropylène glycol.

**27.** Procédé de traitement cosmétique de la peau destiné à lui conférer une coloration artificielle proche du bronzage naturel, **caractérisé en ce qu'**il consiste à appliquer sur celle-ci une quantité efficace d'une composition telle que défini à l'une quelconque des revendications 1 à 28.

**28.** Utilisation d'au moins un agent filtrant les radiations UV, organique ou minéral et d'au moins un composé du type sel de flavylium tels que définis à l'une quelconque des revendications précédentes dans des, ou pour la fabrication de, compositions cosmétiques et/ou dermatologiques dans le but de conférer à la peau une coloration artificielle proche du bronzage naturel.

**Claims**

**1.** Cosmetic and/or dermatological composition intended to give the skin an artificial coloration close to that of a natural tan, **characterized in that** it comprises, in a cosmetically acceptable support, at least one organic or inorganic agent for screening out UV radiation and at least one flavylium salt compound which corresponds to the formula (I) below:

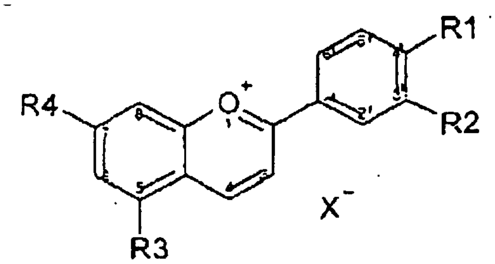

(I)

in which formula (I):

- $R_1$ denotes an OH or a linear or branched, saturated or unsaturated $C_1$-$C_8$ alkoxy radical,
- $R_2$, $R_3$ and $R_4$, which may be identical or different, denote H or $R_1$, it being understood that at least one of the radicals $R_1$ to $R_4$ denotes OH,
- $X^-$ is an organic or inorganic anion and preferably a halide or an organic acid derivative, obtained synthetically or from a plant extract containing it or else from an enriched plant extract.

**2.** Composition according to Claim 1, **characterized in that** it gives, 30 minutes after application to a fair skin at a rate of 2 mg/cm$^2$, a darkening **characterized in** the L*a*b* colorimetric measuring system by a ΔL* ranging from -0.5 to -20.

**3.** Composition according to Claim 2, in which the ΔL* ranges from -0.5 to -15.

**4.** Composition according to Claims 1 to 3, **characterized in that** it produces, 30 minutes after application to a fair skin at a rate of 2 mg/cm$^2$, a coloration defined in the L*a*b* colorimetric system by a ratio Δa*/Δb* ranging from 0.3 to 3.

**5.** Composition according to Claim 3, in which the ratio Δa*/Δb* ranges from 0.8 to 2.

**6.** Composition according to Claim 1, in which, in formula (I), $R_1$ denotes OH or $OCH_3$.

**7.** Composition according to Claim 5, in which the compound of formula (I) is chosen from the following chlorides:

- 4',5,7-trihydroxyflavylium salt (apigeninidine chloride),
- 3',4',7-trihydroxyflavylium salt,
- 4'-hydroxyflavylium salt,
- 4',7-dihydroxyflavylium salt,
- 3',4'-dihydroxyflavylium salt,
- 3',4'-dihydroxy-7-methoxyflavylium salt,
- 3',4',5,7-tetrahydroxyflavylium salt,
- 3',4',5',5,7-pentahydroxyflavylium salt.

**8.** Composition according to Claim 7, **characterized in that** it is the chloride of the 4',5',7-trihydroxy flavylium salt in pure form which may be obtained synthetically.

**9.** Composition according to Claim 7, **characterized in that** it is the chloride of the 4',5,7-trihydroxy flavylium salt, in the form of a plant extract.

**10.** Composition according to Claim 9, **characterized in that** the plant extract is a plant extract obtained from leaves of Sorghum caudatum, from stems, seeds or leaves of Sorghum bicolor, from the petals of Gesneria fulgens, and also from the species Blechum procerum and Sorghum in combination with Colletotrichum graminicola.

**11.** Composition according to Claim 9, **characterized in that** the plant extract is an extract of Sorghum bicolor which may be obtained by an acidic aqueous-alcoholic extraction at an extraction temperature ranging from 30°C to 40°C with a ratio of the volume of solvent to the mass of Sorghum bicolor leaves ranging from 10 to 30.

**12.** Composition according to Claim 11, **characterized in that** the extract of Sorghum bicolor has a titre of from 0.05% to 50% by weight of chloride of the 4',5,7-trihydroxy flavylium salt.

**13.** Composition according to any one of Claims 1 to 11, in which the concentration of flavylium salt compound ranges from 0.0001% to 10%.

**14.** Composition according to Claim 13, in which the concentration of flavylium salt compound ranges from 0.001% to 5% by weight relative to the total weight of the composition.

**15.** Composition according to any one of Claims 1 to 14, in which the organic agent(s) for screening out UV radiation is (are) water-soluble, liposoluble or insoluble in the usual cosmetic solvents.

**16.** Composition according to any one of Claims 1 to 15, in which the organic agent(s) for screening out UV radiation is (are) chosen from anthranilates; cinnamic derivatives; dibenzoylmethane derivatives; salicylic derivatives; camphor derivatives; triazine derivatives; benzophenone derivatives; $\beta,\beta'$-diphenylacrylate derivatives, benzotriazole derivatives, benzimidazole derivatives; imidazolines; bis-benzazolyl derivatives; p-aminobenzoic acid (PABA) derivatives; methylenebis(hydroxyphenylbenzotriazole) derivatives; screening polymers and screening silicones; dimers derived from $\alpha$-alkylstyrene; 4,4-diarylbutadiene derivatives.

**17.** Composition according to Claim 16, in which the organic agent(s) for screening out UV radiation is (are) chosen from:

- Ethylhexyl salicylate,
- Butylmethoxydibenzoylmethane,
- Ethylhexyl methoxycinnamate,
- Octocrylene,
- Phenylbenzimidazolesulphonic acid,
- Terephthalylidene dicamphorsulphonic acid,
- Benzophenone-3,
- Benzophenone-4,
- Benzophenone-5,

- 4-Methylbenzylidenecamphor,
- Benzimidazilate,
- Anisotriazine,
- Ethylhexyltriazone,
- Diethylhexylbutamidotriazone,
- Methylenebis(benzotriazolyl)tetramethylbutylphenol,
- Drometrizole trisiloxane,

and mixtures thereof.

**18.** Composition according to any one of Claims 1 to 15, in which the inorganic agent(s) for screening out UV radiation is (are) chosen from coated or uncoated metal oxide pigments and nanopigments.

**19.** Composition according to Claim 16, in which the inorganic agent(s) for screening out UV radiation is (are) chosen from nanopigments of titanium oxide, of iron oxide, of zinc oxide, of zirconium oxide or of cerium oxide, which may be coated or uncoated.

**20.** Composition according to any one of Claims 1 to 19, in which the agent(s) for screening out UV radiation is (are) present in proportions ranging from 0.1% to 15% by weight relative to the total weight of the composition.

**21.** Composition according to Claim 20, in which the agent(s) for screening out UV radiation is (are) present in proportions ranging from 0.1% to 15% by weight relative to the total weight of the composition.

**22.** Composition according to any one of Claims 1 to 21, containing at least 5% by weight, relative to the weight of the composition, of one or more polyhydroxylated solvents.

**23.** Composition according to Claim 22, in which the polyhydroxylated solvents are chosen from glycols and glycol ethers.

**24.** Composition according to Claim 22 or 23, in which the polyhydroxylated solvents are chosen from ethylene glycol, propylene glycol, butylene glycol, dipropylene glycol and diethylene glycol, and mixtures thereof.

**25.** Composition according to any one of Claims 22 to 24, containing a mixture of three different polyhydroxylated solvents.

**26.** Composition according to Claim 25, containing a mixture consisting of propylene glycol, of butylene glycol and of dipropylene glycol.

**27.** Cosmetic process for treating the skin which is intended to give it an artificial coloration close to that of a natural tan, **characterized in that** it consists in applying to the skin an effective amount of a composition as defined in any one of Claims 1 to 26.

**28.** Use of at least one organic or inorganic agent for screening out UV radiation and of at least one flavylium salt compound, as defined in any one of the preceding claims, in or for the manufacture of cosmetic and/or dermatological compositions with the aim of giving the skin an artificial coloration close to that of a natural tan.

**Patentansprüche**

**1.** Kosmetische und/oder dermatologische Zusammensetzung zur künstlichen Färbung der Haut in einer Farbe, die der natürlichen Bräunung nahe kommt, **dadurch gekennzeichnet, dass** sie in einem kosmetisch akzeptablen Träger mindestens ein organisches oder anorganisches UV-Filter und mindestens eine Verbindung vom Typ der Flavyliumsalze enthält, die auf synthetischem Wege hergestellt oder aus einem Pflanzenextrakt oder einem angereicherten Pflanzenextrakt, der sie enthält, gewonnen wird, wobei die Verbindung vom Typ der Flavyliumsalze der folgenden Formel (I) entspricht:

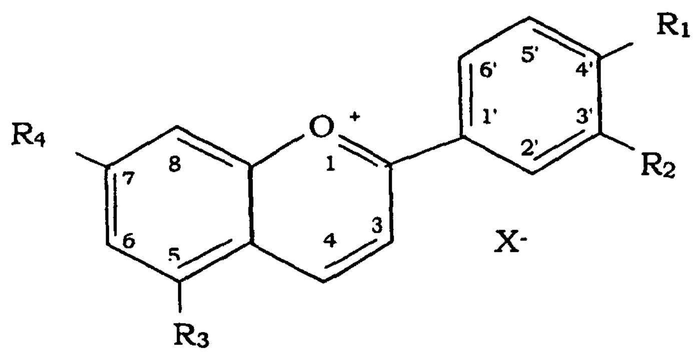

(I)

worin bedeuten:

- $R_1$ die OH-Gruppe oder eine geradkettige oder verzweigte, gesättigte oder ungesättigte $C_{1-8}$-Alkoxygruppe,
- $R_2$, $R_3$ und $R_4$, die gleich oder verschieden sind, H oder $R_1$, mit der Maßgabe, dass mindestens eine der Gruppen $R_1$ bis $R_4$ OH bedeutet,
- $X^-$ ein organisches oder anorganisches Anion und vorzugsweise ein Anion vom Halogenidtyp oder ein von einer anorganischen Säure abgeleitetes Anion.

2. Zusammensetzung nach Anspruch 1, **dadurch gekennzeichnet, dass** 30 Minuten nach dem Auftragen der Zusammensetzung in einer Menge von 2 $mg/cm^2$ auf helle Haut ein Dunklerwerden der Hautfarbe erreicht wird, das im colorimetrischen L* a* b*-System durch ein $\Delta L^*$ von -0,5 bis -20 gekennzeichnet ist.

3. Zusammensetzung nach Anspruch 2, **dadurch gekennzeichnet, dass** $\Delta L^*$ im Bereich von -0,5 bis -15 liegt.

4. Zusammensetzung nach einem der Ansprüche 1 bis 3, **dadurch gekennzeichnet, dass** sie 30 Minuten nach dem Auftragen auf einen hellen Hauttyp in einer Menge von 2 $mg/cm^2$ zu einer Färbung führt, die im colorimetrischen System L*, a*, b* durch ein Verhältnis $\Delta a^*/\Delta b^*$ von 0,3 bis 3 definiert ist.

5. Zusammensetzung nach Anspruch 4, **dadurch gekennzeichnet, dass** das Verhältnis $\Delta a^*/\Delta b^*$ im Bereich von 0,8 bis 2 liegt.

6. Zusammensetzung nach Anspruch 1, wobei in der Formel (1) $R_1$ OH oder $OCH_3$ bedeutet.

7. Zusammensetzung nach Anspruch 5, wobei die Verbindung der Formel (1) unter den folgenden Chloriden ausgewählt ist:

- 4',5,7-Trihydroxyflavyliumchlorid (oder Apigeninidinchlorid),
- 3',4',7-Trihydroxyflavyliumchlorid,
- 4'-Hydroxyflavyliumchlorid,
- 4',7-Dihydroxyflavyliumchlorid,
- 3',4'-Dihydroxyflavyliumchlorid,
- 3',4'-Dihydroxy-7-methoxyflavyliumchlorid,
- 3',4',5,7-Tetrahydroxyflavyliumchlorid,
- 3',4',5',5,7-Pentahydroxyflavyliumchlorid.

8. Zusammensetzung nach Anspruch 7, **dadurch gekennzeichnet, dass** es sich um das 4',5,7-Trihydroxyflavyliumchlorid in reiner Form handelt, das auf synthetischem Wege erhältlich ist.

9. Zusammensetzung nach Anspruch 7, **dadurch gekennzeichnet, dass** es sich um das 4',5,7-Trihydroxyflavyliumchlorid in Form eines Pflanzenextrakts handelt.

10. Zusammensetzung nach Anspruch 9, **dadurch gekennzeichnet, dass** der Pflanzenextrakt ein Pflanzenextrakt ist, der aus den Blättern von Sorghum caudatum; Stängeln, Samen oder Blättern von Sorghum Bicolor; Blütenblättern von Gesneria Fulgens sowie den Arten Blechum Procerum und Sorgho in Kombination mit Colletotrichum

Graminicola erhalten wird.

11. Zusammensetzung nach Anspruch 9, **dadurch gekennzeichnet, dass** der Pflanzenextrakt ein Extrakt von Sorghum Bicolor ist, der durch eine wässerig-alkoholische saure Extraktion bei einer Extraktionstemperatur von 30 bis 40 °C mit einem Volumenverhältnis Lösungsmittel/Masse der Blätter von Sorghum Bicolor von 10 bis 30 erhältlich ist.

12. Zusammensetzung nach Anspruch 11, **dadurch gekennzeichnet, dass** der Extrakt von Sorghum Bicolor 0,05 bis 50 Gew.-% 4',5,7-Trihydroxyflavyliumchlorid enthält.

13. Zusammensetzung nach einem der Ansprüche 1 bis 11, wobei die Konzentration der Verbindung vom Typ der Flavyliumsalze im Bereich von 0,0001 bis 10 %, bezogen auf das Gesamtgewicht der Zusammensetzung, liegt.

14. Zusammensetzung nach Anspruch 13, wobei die Konzentration der Verbindung vom Typ der Flavyliumsalze im Bereich von 0,001 bis 5 Gew.-%, bezogen auf das Gesamtgewicht der Zusammensetzung, liegt.

15. Zusammensetzung nach einem der Ansprüche 1 bis 14, wobei die organischen UV-Filter, wasserlöslich, fettlöslich oder in üblichen kosmetischen Lösungsmitteln unlöslich sind.

16. Zusammensetzung nach einem der Ansprüche 1 bis 15, wobei die organischen UV-Filter unter den Anthranilaten; Zimtsäurederivaten; Dibenzoylmethanderivaten; Salicylsäurederivaten; Campherderivaten; Triazinderivaten; Benzophenonderivaten; β,β'-Diphenylacrylatderivaten, Benzotriazolderivaten, Benzimidazolderivaten; Imidazolinderivaten; Bis-benzoazolylderivaten; p-Aminobenzoesäurederivaten (PABA); Methylen-bis(hydroxyphenylbenzotriazol)derivaten; Filterpolymeren und Filtersiliconen; Dimeren, die von α-Alkylstyrolderivaten abgeleitet sind; und 4,4-Diarylbutadienderivaten ausgewählt sind.

17. Zusammensetzung nach Anspruch 16, wobei die organischen UV-Filter ausgewählt sind unter:

- Ethylhexyl Salicylate,
- Butyl Methoxydibenzolymethane,
- Ethylhexyl Methoxycinnamate,
- Octocrylene,
- Phenylbenzimidazole Sulfonic Acid,
- Terephthalylidene Dicamphor Sulfonic Acid,
- Benzophenone-3,
- Benzophenone-4,
- Benzophenone-5,
- 4-Methylbenzylidene camphor,
- Benzimidazilate,
- Anisotriazine,
- Ethylhexyl triazone,
- Diethylhexyl Butamido Triazone,
- Methylene bis-Benzotriazolyl Tetramethylbutylphenol,
- Drometrizone Trisiloxane, und
- deren Gemischen.

18. Zusammensetzung nach einem der Ansprüche 1 bis 15, wobei die anorganischen UV-Filter unter den Pigmenten und Nanopigmenten von Metalloxiden ausgewählt sind, die umhüllt oder nicht umhüllt vorliegen.

19. Zusammensetzung nach Anspruch 16, wobei der oder die anorganischen UV-Filter unter den Nanopigmenten von Titanoxid, Eisenoxid, Zinkoxid, Zirconiumoxid und Ceroxid ausgewählt sind, die umhüllt oder nicht umhüllt vorliegen.

20. Zusammensetzung nach einem der Ansprüche 1 bis 21, wobei der oder die UV-Filter in Mengenanteilen von 0,1 bis 15 Gew.-%, bezogen auf das Gesamtgewicht der Zusammensetzung, vorliegen.

21. Zusammensetzung nach Anspruch 22, wobei der oder die UV-Filter in Mengenanteilen von 0,1 bis 15 Gew.-%, bezogen auf das Gesamtgewicht der Zusammensetzung, vorliegen.

**22.** Zusammensetzung nach einem der Ansprüche 1 bis 23, die mindestens 5 Gew.-% eines oder mehrerer polyhydroxylierter Lösungsmittel, bezogen auf das Gewicht der Zusammensetzung, enthält.

**23.** Zusammensetzung nach Anspruch 24, wobei die polyhydroxylierten Lösungsmittel unter den Glykolen und Glykolethern ausgewählt sind.

**24.** Zusammensetzung nach Anspruch 24 oder 25, wobei die polyhydroxylierten Lösungsmittel unter Ethylenglykol, Propylenglykol, Butylenglykol, Dipropylenglykol oder Diethylenglykol und deren Gemischen ausgewählt sind.

**25.** Zusammensetzung nach einem der Ansprüche 24 bis 26, die ein Gemisch von mindestens drei verschiedenen polyhydroxylierten Lösungsmitteln enthält.

**26.** Zusammensetzung nach Anspruch 27, die ein Gemisch enthält, das aus Propylenglykol, Butylenglykol und Dipropylenglykol besteht.

**27.** Verfahren zur kosmetischen Behandlung der Haut, das dazu dient, der Haut eine künstliche Färbung zu geben, die der natürlichen Bräunung nahe kommt, **dadurch gekennzeichnet, dass** es darin besteht, auf die Haut eine wirksame Menge einer Zusammensetzung nach einem der Ansprüche 1 bis 28 aufzutragen.

**28.** Verwendung mindestens eines organischen oder anorganischen UV-Filters und mindestens einer Verbindung vom Typ der Flavyliumsalze nach einem der vorhergehenden Ansprüche in kosmetischen und/oder dermatologischen Zusammensetzungen, um der Haut eine künstliche Färbung zu geben, die der natürlichen Bräunung nahe kommt.